# EUROPEAN PATENT SPECIFICATION

(11) **EP 2 642 973 B1**
(45) Date of publication and mention of the grant of the patent: **21.03.2018**
(21) Application number: 11785405.9
(22) Date of filing: 17.11.2011
(51) Int. Cl.: A61K 8/34, A61K 8/40, A61Q 19/02, A61Q 17/04, A61K 8/35

(54) **COSMETIC COMPOSITION FOR SKIN WHITENING COMPRISING RESVERATROL**
KOSMETISCHE ZUSAMMENSETZUNG MIT RESVERATROL ZUR HAUTAUFHELLUNG
COMPOSITION COSMÉTIQUE POUR LE BLANCHIMENT DE LA PEAU COMPRENANT DU RESVÉRATROL

(30) Priority: 22.11.2010 EP 10014848
(43) Date of publication of application: 02.10.2013
(73) Proprietor: DSM IP Assets B.V., 6411 TE Heerlen (NL)
(72) Inventor: BECK, Mareike, CH-4002 Basel (CH); DEN BRAVE, Kerstin, CH-4002 Basel (CH); FLORES-CANDIA, Juana-Lucia, CH-4002 Basel (CH); LU, Yingzi, CH-4002 Basel (CH)
(74) Representative: Berg, Katja
(86) International application number: PCT/EP2011/070392
(87) International publication number: WO 2012/069362

(56) References cited:
- WO-A1-2009/129627
- WO-A2-2004/103265

## Description

One important aspect of the present invention is the use of octocrylene and avobenzone, if desired in combination with polyols to enhance the dermal penetration of resveratrol in topical cosmetic compositions, particularly compositions for skin whitening.

Resveratrol is UV sensitive and poorly soluble in cosmetic solvents which may reduce its stability and efficacy in day care cosmetic compositions. It has been found that the combination of resveratrol with UV-filters and, if desired, polyols shows the following benefits:
a) prevents or reduces UV-induced resveratrol breakdown,
b) enhances dermal penetration, thus improving bio-availability
c) avoids recrystallization of resveratrol and
d) enhances stability of the cosmetic formulation.

The term "breakdown" relates to any chemical change which trans-resveratrol may undergo, e.g., oxidation, hydrogenation and, particularly, isomerization, having a negative impact on its biological activities.

One of the most important areas of the skin care market is related to cosmetic compositions with enhanced skin whitening properties as consumers are expressing strong interest in achieving uniform and lighter skin tone. Solar lentigos, post-inflammatory hyperpigmentation and melasma are skin disorders widely distributed in human population. Furthermore, skin lightening is one of the cosmetic market segments showing the biggest growth; driven largely by expanding Asian markets, as well as by extension of skin whitening products to specific consumer segments (i.e., men care). Different products exhibiting skin whitening activities exist in the market (e.g., ascorbyl glucoside, arbutins, plant extracts, kojic acid, Vitamin C derivatives), however these often show formulation or penetration constraints, have low in-vivo efficacy and/or give rise to safety concerns. As consumers are becoming increasingly aware of the toxicity issues related to some of these whitening agents, effective and safe whitening actives are sought.

Resveratrol, a naturally occurring molecule found, e.g., in giant knotweed, grapes and, consequently, in red wine has been the subject of intense research in recent years. Scientific reports are increasingly demonstrating the multi-functional benefits of resveratrol. Resveratrol is reported to be an extremely potent anti-oxidant, a modulator of genetic expression via signal transduction, an inhibitor of inflammatory mediators, to have phytohormonal benefits, and to reduce the synthesis of melanine. Such combination of biological functions and the cosmetic effects makes resveratrol a unique active ingredient for personal care products.

JP 64-38009, published February 8, 1989, discloses skin-lightening cosmetic composition on the basis alone of one or more hydroxystilbenes, e.g. resveratrol, at concentrations of 0.00001 to 10 wt-%. The compositions comprise usual cosmetic adjuvants and additives/ carriers, such as oils, preservatives, perfumes, and emulsifiers; e.g., polyethylene glycol (PEG).

WO2004103265 discloses a stabilised skin-whitening and antioxidative composition comprising resveratrol and a stabiliser such as polyethylene glycol.

WO2009129627 discloses dimethyl isosorbide as a solubiliser/stabilizer for resveratrol.

Despite all the above biological properties and its superior skin whitening effects, resveratrol poses a set of challenges when developing cosmetic compositions. Due to its poor solubility it tends to precipitate as a crystal in cosmetic compositions containing water. High content reveratrol is believed to be feasible only in a substantially water-free cosmetic composition. Little is known about skin penetration of resveratrol as a function of a given cosmetic composition. Published data on the chemical stability of *trans*-resveratrol (the active form) have varied greatly. There are different views on the conditions affecting cis/trans formation.

Accordingly it was an objective to find a whitening cosmetic composition containing resveratrol wherein the resveratrol in its stabilized active form can penetrate into the skin in sufficient amount as well as a method for the preparation of such compositions.

In accordance with the present invention it has been found that the use of octocrylene and avobenzone, if desired in combination with one or more polyols in topical cosmetic compositions, particularly for skin whitening, comprising resveratrol is advantageous. In such compositions res-veratrol, particularly the trans-isomer, is stabilized, can better penetrate the skin and shows an improved whitening effect.

According to the present invention the amount of resveratrol in the composition is in the range of from 0.001 to 5 % by weight, preferably in the range of from 0.05 to 2 % by weight, most preferably in the range of from 0.2 to 1 % by weight, each with respect to the total weight of the composition.

The UV-filters according to the present invention are butylmethoxydibenzoylmethane and octocrylene.

The amount of UV-filter substances employed can vary depending upon the degree of desired protection from UV-radiation and upon the level of trans-resveratrol used in the cosmetic composition. It is usually in the range of from 4 to 27 % by weight, preferably in the range of from 6 to 20 % by weight, most preferably in the range of from 9 to 15 % by weight, each with respect to the total weight of the composition.

The weight ratio of the UV-filter substance(s) to resveratrol is lower than 150 : 1, preferably from 100 : 1 to 10 : 1, most preferably from 50 : 1 to 10 :1.

The polyols in accordance with the present invention are those non-aqueous polar organic solvents which are capable of solubilizing resveratrol. Among the polyols, which are linear and branched chain alkyl polyhydroxyl compounds, propylene glycol, sorbitol, butylene glycol, and glycerin are illustrative examples. Specially preferred are polymeric polyols such as poly-propylene glycol, polyethylene glycol and derivatives thereof. Examples of polymeric polyols include PEG-18, PPG-18, dimethicone, PEG-40 hydrogenated castor oil, PEG-20 stearate, PEG-20 methyl glucose sesquistearate, PEG-120 methyl glucose dioleate, ceteareth-12, coceth-7, PPG-1-PEG-9 lauryl glycol ether, PEG-30 glyceryl stearate, PEG-7 glyceryl cocoate and ethoxyglycol.

According to the present invention the amount of one or more polyols in the composition is in the range of from 1 to 30 % by weight, preferably in the range of from 3 to 20 % by weight, most preferably in the range of from 5 to 17 % by weight, each with respect to the total weight of the composition.

The weight ratio of one or more polyols to resveratrol in the compositions of the present invention is lower than 50 : 1, preferably in the range from 30 : 1 to 20 : 1, more preferably in the range from 15 : 1 to 5 : 1.

The topical cosmetic compositions of the invention can also contain usual cosmetic adjuvants and additives, such as preservatives/antioxidants, fatty substances/oils, water, organic solvents, silicones, thickeners, softeners, emulsifiers, sunscreens, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sen-sates, astringents, antifoaming agents, pigments or nanopigments or any other ingredients usually formulated into cosmetic compositions. Such cosmetic ingredients commonly used in the skin care industry which are suitable for use in the compositions of the present invention are, e.g., described in the CTFA Cosmetic Ingredient Handbook, Second Edition (1992) without being limited thereto.

The term "topical composition" as used herein refers to a cosmetic composition that can be topically applied to mammalian keratinous tissue. The term "cosmetic preparation" or "cosmetic composition" as used in the present application refers to cosmetic compositions as defined under the heading "Kosmetika" in Römpp Lexikon Chemie, 10th edition 1997, Georg Thieme Verlag Stuttgart, New York.

Preferably, the topical compositions according to the present invention are in the form of a suspension or dispersion in solvents or fatty substances, or alternatively in the form of an emulsion or micro emulsion (in particular of O/W- or W/O-type), PIT-emulsion, multiple emulsion (e. g. O/W/O- or W/O/W-type), pickering emulsion, hydrogel, alcoholic gel, lipogel, one- or multiphase solution or vesicular dispersion or other usual forms, which can also be applied by pens, as masks or as sprays. If the topical composition is or comprises an emulsion it can also contain one or more anionic, nonionic, cationic or amphoteric surfactant(s). Preferred are emulsions of the oil-in-water, water-in-oil or silicone-water type, nanoemulsions, microemulsions, multiple emulsions, aqueous or anhydrous gels and solutions. Most preferred are O/W-emulsions and/or gels.

Preferred topical compositions according to the invention are skin care preparations, decorative preparations, light protection preparations and functional preparations.

Examples of skin care preparations are, in particular, face creams, body oils, body lotions, body gels, treatment creams, skin protection ointments, shaving preparations, such as shaving foams or gels, skin powders such as baby powder, moisturizing gels, moisturizing sprays, revitalizing body sprays, cellulite gels, face and/or body moisturizers, facial and/or body cleansers, face masks, anti acne preparations and/or peeling preparations. Most preferred are face care products.

Examples of decorative preparations are, in particular, lipsticks, eye shadows, mascaras, dry and moist make-up formulations, rouges, powders, and/or suntan lotions.

Examples of functional preparations are cosmetic compositions containing further active ingredients such as hormone preparations, vitamin preparations, vegetable and/or fruit extracts, anti-ageing preparations, and/or antimicrobial (antibacterial or antifungal) preparations without being limited thereto.

Topical compositions in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foam, a spray, a stick, a plaster, a cleanser, a soap or a wipe.

In accordance with the present invention, the topical composition may preferably contain one or more further cosmetically active ingredient(s), in particular for skin lightening; tanning prevention; treatment of hyperpigmentation; preventing or reducing acne, wrinkles, lines, atrophy and/or inflammation; as well as topical antimicrobial and/or antifungal agents; chelators and/or sequestrants; anti-cellulites agents (e.g. phytanic acid) and/or carriers and/or excipients or diluents conventionally used in topical compositions. The necessary amounts of the cosmetic and dermatological adjuvants and additives can, based on the desired product, easily be determined by the skilled person.

The invention is explained in more detail by the following description of specific compositions and of experiments.

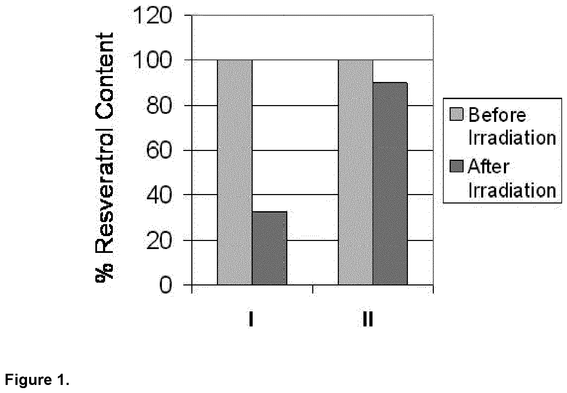

**Figure 1****:** Effect of UV-irradiation on trans-resveratrol content in two cosmetic carriers without (I) and with (II) UV-Filters.

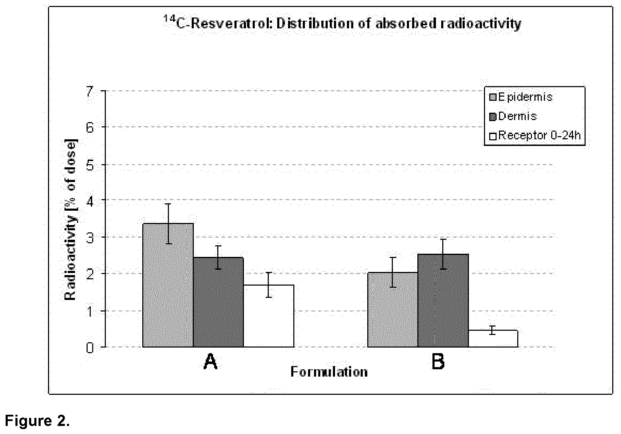

**Figure 2****:** Dermal penetration of Resveratrol on standard O/W cosmetic composition as a function of the active ingredient level **(A) 0.01 % resveratrol** (4 donors, 13 replicates, recovery 97.5 % ± 6.2 %) and **(B) 0.05 % resveratrol** (4 donors, 12 replicates, recovery 92.1 % ± 6.3 %). Penetration is depicted as % of dose applied.

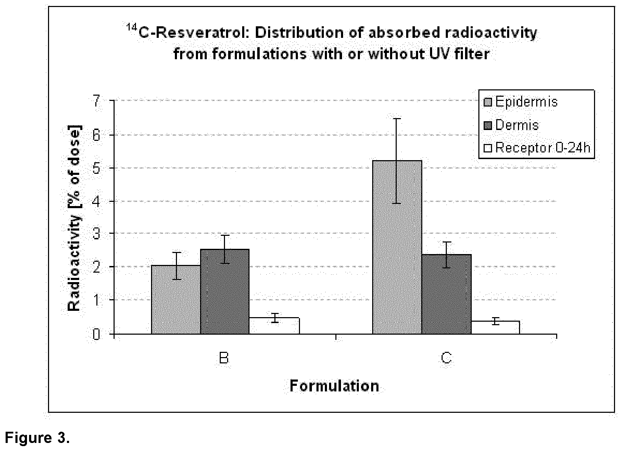

**Figure 3****:** Impact of UV-filters on resveratrol dermal penetration. **(B)** O/W cosmetic composition with **0.05 % resveratrol** (4 donors, 12 replicates, recovery 92.1 % ± 6.3 %); and **(C)** O/W cosmetic composition containing **UV-filters** and **0.05 % resveratrol** (4 donors, 8 replicates, recovery 97.3 % ± 2.7 %). Penetration is depicted as % of dose applied.

### Examples

### Stability of Resveratrol

It has been observed that cosmetic compositions containing resveratrol, independent of the pH, develop dark coloration upon exposure to day light under long term storage conditions. This observation has prompted a closer look into the photo-stability of resveratrol. To this end, 1 weight-% of resveratrol was dissolved in two different cosmetic carriers:
I) Mirytol (29 weight-%) and isopropanol (70 weight-%);
II) Mirytol (29 weight-%), butylmethoxydibenzoylmethane (4 weight-%), octocrylene (10 weight-%) and isopropanol (70 weight-%).

6 x 20 ul of cosmetic solutions I and II each were distributed on glass plates and irradiated with 10 MED. Resveratrol content was determined after irradiation by HLPC.

As shown by Figure 1, it has been found that about 70 % of the initial trans-resveratrol was lost due to UV-irradiation in the cosmetic carrier (I). Such loss was reduced from nearly 70 % to 10 % when using UV-Filter in solution (II) as shown.

### Solubility, compatibility and stability of trans-resveratrol in cosmetic compositions containing UV-filters

Resveratrol was formulated in cosmetic compositions depicted in Tables 1 and Table 2 in an attempt to evaluate its stability and compatibility with organic and inorganic UV-filters. Avobenzone and octocrylene were selected from the organic UV-filters as these are the most widely used UV-Filters in day care cosmetic compositions. From the inorganic UV-filters, a coated form or titanium dioxide was selected.

**Table 1. Cosmetic composition containing resveratrol and organic UV-Filters. Preparation consisted in heating phase A and phase B, adding phase B to A, homogenizing the emulsion and adding phase C after the emulsion reached room temperature.**

| **Phase Ingredients** | | **INCI Name** | **% w / w** |
|---|---|---|---|
| A | Dermofeel BGC | Butylene glycol dicaprylate/dicaprate | 3.00 |
| | PARSOL® 1789 | Butyl-methoxydibenzoylmethane (Avobenzone; USAN) | 4.50 |
| | PARSOL® 340 | Octocrylene (Octocrilene; USAN) | 4.00 |
| | Eutanol G | Octyldodecanol | 3.00 |
| | Cetiol OE | Dicaprylylether | 3.00 |
| | Tinosorb S | Bis-ethylhexyloxyphenol Methoxyphenyl triazine | 2.00 |
| | Cetiol CC | Dicaprylyl carbonate | 2.00 |
| | Imwitor 372 P Schuppen | Glyceryl stearate citrate | 1.00 |
| | Lanette 18 | Stearyl alcohol | 1.00 |
| | Lipocire Na 10 Pastilles | Hydrogenated coco-glycerides | 1.00 |
| | dl-alpha -Tocopheryl Acetate | Tocopheryl acetate | 0.50 |
| | Antaron V-216 | VP/Hexadecene copolymer | 1.00 |
| | Butylated Hydroxytoluene | BHT | 0.05 |
| | Phenonip | Phenoxyethanol, methylparaben, ethylparaben, butylparaben, propylparaben, isobutylparaben | 0.80 |
| | Finsolv TN | C12-15 Alkyl Benzoate | 5.00 |
| | **Resveratrol** | **trans-Resveratrol** | 0.05 |
| | | | |
| B | Glycerin | Glycerin | 8.00 |
| | Keltrol CG-T | Xanthan gum | 0.30 |
| | Pemulen TR-1 | Acrylates/C10-30 Alkyl Acrylate Crosspolymer | 0.30 |
| | Edeta BD | Disodium EDTA | 0.10 |
| | Water dem. | Aqua | 55.05 |
| | | | |
| C | Ethanol | Alcohol | 4.00 |
| | Triethanolamine (T.E.A.) | Triethanolamine | 0.35 |

**Table 2. Cosmetic composition containing resveratrol and a combination of organic and inorganic UV-Filters. Preparation consisted in heating phase A and phase B, adding phase B to A, homogenizing the emulsion and adding phase C after the emulsion reached room temperature.**

| **Phase Ingredients** | | **INCI Name** | **% w / w** |
|---|---|---|---|
| A | Dermofeel BGC | Butylene glycol dicaprylate/dicaprate | 3.00 |
| | PARSOL® 1789 | Butyl-methoxydibenzoylmethane (Avobenzone; USAN) | 4.50 |
| | PARSOL® 340 | Octocrylene (Octocrilene; USAN) | 4.00 |
| | Eutanol G | Octyldodecanol | 3.00 |
| | Cetiol OE | Dicaprylyl ether | 3.00 |
| | Tinosorb S | Bis-Ethylhexyloxyphenol methoxyphenyl triazine | 2.00 |
| | Cetiol CC | Dicaprylyl carbonate | 2.00 |
| | Imwitor 372 P Schuppen | Glyceryl stearate citrate | 1.00 |
| | Lanette 18 | Stearyl alcohol | 1.00 |
| | Lipocire Na 10 Pastilles | Hydrogenated coco-glycerides | 1.00 |
| | dl-alpha -Tocopheryl Acetate | Tocopheryl acetate | 0.50 |
| | Antaron V-216 | VP/Hexadecene copolymer | 1.00 |
| | Butylated Hydroxytoluene | BHT | 0.05 |
| | Phenonip | Phenoxyethanol, methylparaben, ethylparaben, butylparaben, propylparaben, isobutylparaben | 0.80 |
| | PARSOL® TX | Titanium dioxide & dimethicone & silica | 5.00 |
| | **Resveratrol** | **trans-Resveratrol** | 0.05 |
| | Finsolv TN | C12-15 Alkyl benzoate | 5.00 |
| | | | |
| B | Glycerin | Glycerin | 8.00 |
| | Keltrol CG-T | Xanthan gum | 0.30 |
| | Pemulen TR-1 | Acrylates/C10-30 alkyl acrylate crosspolymer | 0.30 |
| | Edeta BD | Disodium EDTA | 0.10 |
| | Water dem. | Aqua | 50.05 |
| | | | |
| C | Ethanol | Alcohol | 4.00 |
| | Triethanolamine (T.E.A.) | Triethanolamine | 0.35 |

As shown in Table 3, resveratrol is compatible with organic and inorganic sun screens. It is solved well in both formulations tested. Neither crystals nor discoloration was observed after long term storage under wide range of temperature. These formulations were stable as judged by the trans-resveratrol content depicted under different storage conditions.

**Table 3. Stability of resveratrol compositions under different storage conditions.**

| | **Measured trans-resveratrol content [%]** | | | |
|---|---|---|---|---|
| **Formulation** | **Day 0** | **Day 94 5 °C** | **Day 94 Room Temp.** | **Day 94 43 °C** |
| **Formulation A** Organic UV-Filters | 0.06 | 0.07 | 0.065 | 0.06 |
| Avobenzone + Octocrylene | | | | |
| **Formulation B** Organic + Inorganic | 0.05 | 0.05 | 0.04 | 0.05 |
| Avobenzone + Octocrylene + Titanium dioxide | | | | |

### Skin penetration of resveratrol

The *in vitro* percutaneous penetration of resveratrol was determined using freshly excised pig ear skin (dermatomed, ca 300 µm thickness). The skin penetration of [phenyl-¹⁴C]-resveratrol was investigated in a system with flow through diffusion cells.

The test substance was formulated as 0.01 % and 0.05 % O/W creams using Brij-72 and Brij-721 emulsifiers (formulations A and B, Table 5) and as 0.05 % O/W cream containing also UV filters and using emulsifiers Imwitor 372 P and Licocire NA (formulation C, Table 6). Actual resveratrol contents were determined as 0.009 %, 0.055 % and 0.061 % in formulations A, B and C, respectively. Creams were applied at dose levels of 4.3-6.7 mg formulation per cm² skin, at a temperature of 32 °C under non-occluded conditions. Four different donor animals were used per formulation.

Skin integrity was tested by means of ³H₂O permeation measurement, and criteria for skin samples to be included in the subsequent resveratrol permeation experiment were: (a) Kp < 2.5 x 10⁻³ cm/h, and (b) total permeation < 25 % of the dose within 6h. Between 8 and 13 individual diffusion cells qualified and were used per tested formulation.

The penetration of the test substance was determined over 24 h by collecting the receptor solution. Receptor fluid was cell culture medium (DMEM) supplemented with 1 % penicillin/streptomycin and 5 % bovine serum albumin, and collection intervals were 0 - 0.5 h, 0.5 - 1 h, 1 - 3 h, 3 - 5 h, 5 - 7 h, 7 - 9 h, and 9 - 24 h post application for formulation B, and 0 - 3 h, 3 - 6 h, 6 - 12 h, 12 - 18 h, and 18 - 24 h post application for formulations A and C. 24 h after application of the test substance the skin was washed off with mild soap solution, the stratum corneum was removed by tape stripping and the remaining skin was separated into epidermis and dermis. The total radioactivity was determined by liquid scintillation counting in all samples collected.

The studies were conducted according to the procedures indicated by the following internationally accepted guidelines and recommendations:
- OECD 428: Skin Absorption: In vitro Method, adopted 13th April 2004.
- OECD Guidance Document No. 28: Guidance Document for the Conduct of Skin Absorption Studies, ENV/JM/MONO (2004) 2, adopted 5th March 2004.
- SCCP Opinion on Basic Criteria for the in vitro Assessment of Dermal Absorption of Cosmetic Ingredients - updated March 2006, SCCP/0970/06, adopted 28th March 2006.

The overall recovery of radioactivity was 93.6 %, 91.8 %, and 97.3 % of the applied dose for formulations A, B and C, respectively. Most of the radioactivity (> 91 %) could be washed off. Minor amounts of radioactivity could be recovered from stratum corneum, epidermis, dermis and receptor fluid.

**Table 4: Preparation without UV-filter of Base Formulation for O/W Formulation A (0.01 % resveratrol and Formulation B (0.05 % resveratrol**

| ***Phase*** | **Ingredients** | **INCI Name** | **A (% w/w)*** | **B (% w/w)*** |
|---|---|---|---|---|
| A | Estol 3650 | Glyceryl myristate | 2.00 | 2.00 |
| | Lanette 16 | Cetyl alcohol | 2.00 | 2.00 |
| | Brij 72 | Steareth-2 | 2.00 | 2.00 |
| | Brij 721 | Steareth-21 | 2.00 | 2.00 |
| | Butylated Hydroxytoluene | BHT | 0.05 | 0.05 |
| | Phenonip | Phenoxyethanol, methylparaben, ethylparaben, butylparaben, propylparaben, isobutylparaben | 0.80 | 0.80 |
| | Dow corning 200/100 cs | Dimethicone | 0.30 | 0.30 |
| | Finsolv TN | C12-15 Alkyl benzoate | 15.00 | 15.00 |
| B | Keltrol CG-T | Xanthan gum | 0.30 | 0.30 |
| C | Edeta BD | Disodium EDTA | 0.10 | 0.10 |
| | 1.2 Propandiol | Propylene glycol | 4.00 | 4.00 |
| | Water dem. | Aqua | 67.44 | 67.40 |
| D | Resvida in ethanol | | 4.01 | 4.05 |

| | | | | |
|---|---|---|---|---|
| * target composition | | | | |

### Procedure

- Heat part A to 80 °C. When everything is dissolved add part B under stirring.
- Heat part C to 80 °C, add to part A/B and homogenize.
- Cool down the emulsion to room temperature (pH ∼5.1)
- Add part D (¹⁴C-resveratrol in ethanol) and stir over night.

**Table 5: Preparation of O/W Formulation C (0.05 % resveratrol + UV filter)**

| ***Phase*** | **Ingredients** | **INCI Name** | **% w / w*** |
|---|---|---|---|
| A | Dermofeel BGC | Butylene glycol dicaprylate/dicaprate | 3.00 |
| | PARSOL® 1789 | Butyl-methoxydibenzoylmethane (Avobenzone; USAN) | 2.50 |
| | PARSOL® 340 | Octocrylene (Octocrilene; USAN) | 6.00 |
| | Eutanol G | Octyldodecanol | 3.00 |
| | Cetiol OE | Dicaprylyl ether | 3.00 |
| | Cetiol CC | Dicaprylyl carbonate | 2.00 |
| | Imwitor 372 P Schuppen | Glyceryl stearate citrate | 1.00 |
| | Lanette O | Cetearyl alcohol | 1.00 |
| | Lipocire Na 10 Pastilles | Hydrogenated coco-glycerides | 1.00 |
| | dl-alpha -Tocopheryl Acetate | Tocopheryl acetate | 0.50 |
| | Antaron V-216 | VP/Hexadecene copolymer | 1.00 |
| | Butylated Hydroxytoluene | BHT | 0.05 |
| | Phenonip | Phenoxyethanol, methylparaben, ethylparaben, butylparaben, propylparaben, isobutylparaben | 0.80 |
| | Finsolv TN | C12-15 Alkyl benzoate | 5.00 |
| | PARSOL® SLX | Polysilicone-15 | 3.00 |
| | Keltrol CG-T | Xanthan gum | 0.30 |
| | Pemulen TR-1 | Acrylates/C10-30 alkyl acrylate crosspolymer | 0.30 |
| B | Glycerin | Glycerin | 6.00 |
| | Edeta BD | Disodium EDTA | 0.10 |
| | Water dem. | Aqua | 58.00 |
| C | Triethanolamine (T.E.A.) | Triethanolamine | 0.40 |
| D | Resveratrol in ethanol | trans-Resveratrol | 2.05 |

| | | | |
|---|---|---|---|
| target composition | | | |

### Procedure

- Heat part A and part B to 80 °C.
- Add part B to part A under stirring until homogeneity.
- Cool down the emulsion to room temperature.
- Adjust pH to 5-5.5 using part C (triethanolamine)
- Add part D (¹⁴C-resveratrol in ethanol) and stir over night

As shown by Figure 2 and against our expectations, the formulation having lower resvertarol content (0.1 %) showed increased penetration rate, particularly at the epidermis layer. Assessment of the extracts showed that although the radio-purity of both formulations was high, a cis-isomer has been found (Table 6). Interestingly, the formulation (B) having lower epidermis penetration rate has shown double content of cis-isomer, suggesting strong correlation between molecule integrity and penetration rate. The different degree of isomerization could be explained by the extent of light exposure during the preparation of the cream and/or during the extraction procedure in the course of the series of experiments.

To confirm such correlation, the composition containing 0.05 % resveratrol (Formulation B) was enriched with a combination of Avobenzone and Octocrylene (Formulation C). Surprisingly no cis-isomer was detected (Table 6) and which is most surprising; the epidermal penetration of resveratrol was increased by about 250 % (Figure 3).

**Table 6: Analysis of radioactivity in the formulations Specific activity of the ¹⁴C-resveratrol: 902.39 MBq/mmol (3.954 kBq/µg) or 237264 dpm/µg.**

| | **Radioactivity** | **Resveratrol** | | |
|---|---|---|---|---|
| | **[dpm/mg]** | **[µg/mg]** | **trans-isomer (%)** | **cis isomer (%)** |
| **¹⁴C-Resveratrol stock solution** | - | - | 100 | 0 |
| **Formulation A** | 21707 | 0.091 | 86.9 | 13.1 |
| **Formulation B** | 130309 | 0.549 | 73.5 | 26.5 |
| **Formulation C** | 144247 | 0.608 | 100 | 0 |

### Enabling high content of resveratrol in stable cosmetic compositions

As our understanding on the conditions affecting stability and penetration of resveratrol improved, we attempted to increase the content of resveratrol from 0.05 % by a factor of 20. This was accomplished by fine tuning the combination of UV-Filters, PEG/PPG-18/18 dimethicone and propylene glycol as depicted in Table 7. This formulation was shown to be stable longer than six months.

**Table 7.**

| **Phase** | **Ingredients** | **INCl Name** | **% w / w** |
|---|---|---|---|
| A | PARSOL® 1789 | Butyl-methoxydibenzoylmethane (Avobenzone; USAN) | 2.00 |
| | PARSOL® 340 | Octocrylene (Octocrilene; USAN) | 6.00 |
| | Imwitor 372 P Schuppen | Glyceryl stearate citrate | 2.00 |
| | Lanette O | Cetearyl alcohol | 1.50 |
| | Lipocire Na 10 Pastilles | Hydrogenated coco-glycerides | 1.00 |
| | **Resveratrol** | **trans-Resveratrol** | 1.00 |
| | Finsolv TN | C12-15 Alkyl benzoate | 7.00 |
| | Myritol PC | Propylene glycol dicaprylate/dicaprate | 5.00 |
| | d-alpha Tocopheryl Acetate | Tocopheryl acetate | 0.20 |
| B | Keltrol CG-T | Xanthan gum | 0.15 |
| | Pemulen TR-1 | Acrylates/C10-30 alkyl acrylate Crosspolymer | 0.30 |
| | Dow Corning 190 Surfactant | PEG/PPG-18/18 dimethicone | 4.00 |
| | 1,2-Propanediol | Propylene glycol | 5.00 |
| | Water dem. | Aqua | 61.83 |
| D | Phenonip | Phenoxyethanol & methylparaben & Eehylparaben & butylparaben & propylparaben & isobutylparaben | 0.80 |
| | Triethanolamine (T.E.A.) | Triethanolamine | 0.22 |
| | Ethanol | Alcohol | 2.00 |

## Claims

1. Use of UV-filters to enhance dermal penetration of resveratrol in topical cosmetic compositions, wherein the UV-filters are octocrylene, and 1-(4-tert-butylphenyl)-3-(4-methoxyphenyl)-propane-1,3-dione.

2. The use according to claim 1 in topical cosmetic compositions for skin whitening.

3. The use according to one of the preceding claims wherein the cosmetic composition furthermore comprises a polyol selected from the group consisting of sorbitol, butylene glycol, glycerol, poly-propylene glycol, polyethylene glycol, PEG-18, PPG-18, dimethicone, PEG-40 hydrogenated castor oil, PEG-20 stearate, PEG-20 methyl glucose sesquistearate, PEG-120 methyl glucose dioleate, ceteareth-12, coceth-7, PPG-1-PEG-9 lauryl glycol ether, PEG-30 glyceryl stearate, PEG-7 glyceryl cocoate and ethoxyglycol.

4. The use according to claim 3 wherein the polyol is a polyethylene glycol.

5. The use according to anyone of the preceding claims wherein the cosmetic composition is in the form of an O/W-emulsion or a gel.

6. The use according to anyone of the preceding claims wherein the amount of resveratrol in the composition is in the range of from 0.001 to 5 % by weight, preferably in the range of from 0.05 to 2 % by weight, most preferably in the range of from 0.2 to 1 % by weight, each with respect to the total weight of the composition.

7. The use according to anyone of the preceding claims wherein the amount of the UV-filters in the composition is in the range of from 4 to 27 % by weight, preferably in the range of from 6 to 20 % by weight, most preferably in the range of from 9 to 15 % by weight, each with respect to the total weight of the composition.

8. The use according to anyone of claims 3 to 7, wherein the amount of one or more polyols in the composition is in the range of from 1 to 30 % by weight, preferably in the range of from 3 to 20 % by weight, most preferably in the range of from 5 to 17 % by weight, each with respect to the total weight of the composition.

## Patentansprüche

1. Verwendung von UV-Filtern zur Verbesserung der Hautpenetration von Resveratrol in topischen kosmetischen Zusammensetzungen, wobei es sich bei den UV-Filtern um Octocrylen und 1-(4-tert-Butylphenyl)-3-(4-methoxyphenyl)propan-1,3-dion handelt.

2. Verwendung nach Anspruch 1 in topischen kosmetischen Zusammensetzungen zur Hautaufhellung.

3. Verwendung nach einem der vorhergehenden Ansprüche, wobei die kosmetische Zusammensetzung des Weiteren ein Polyol aus der Gruppe bestehend aus Sorbitol, Butylenglykol, Glycerin, Polypropylenglykol, Polyethylenglykol, PEG-18, PPG-18, Dimethicon, PEG-40-hydriertes Rizinusöl, PEG-20-Stearat, PEG-20-Methylglucosesesquistearat, PEG-120-Methylglucosedioleat, Ceteareth-12, Coceth-7, PPG-1-PEG-9-Laurylglykolether, PEG-30-Glycerylstearat, PEG-7-Glycerylcocoat und Ethoxyglykol umfasst.

4. Verwendung nach Anspruch 3, wobei es sich bei dem Polyol um ein Polyethylenglykol handelt.

5. Verwendung nach einem der vorhergehenden Ansprüche, wobei die kosmetische Zusammensetzung in Form einer O/W-Emulsion oder eines Gels vorliegt.

6. Verwendung nach einem der vorhergehenden Ansprüche, wobei die Menge an Resveratrol in der Zusammensetzung im Bereich von 0,001 bis 5 Gew.-%, vorzugsweise im Bereich von 0,05 bis 2 Gew.-%, ganz besonders bevorzugt im Bereich von 0,2 bis 1 Gew.-%, jeweils bezogen auf das Gesamtgewicht der Zusammensetzung, liegt.

7. Verwendung nach einem der vorhergehenden Ansprüche, wobei die Menge der UV-Filter in der Zusammensetzung im Bereich von 4 bis 27 Gew.-%, vorzugsweise im Bereich von 6 bis 20 Gew.-%, ganz besonders bevorzugt im Bereich von 9 bis 15 Gew.-%, jeweils bezogen auf das Gesamtgewicht der Zusammensetzung, liegt.

8. Verwendung nach einem der Ansprüche 3 bis 7, wobei die Menge eines oder mehrerer Polyole in der Zusammensetzung im Bereich von 1 bis 30 Gew.-%, vorzugsweise im Bereich von 3 bis 20 Gew.-%, ganz besonders bevorzugt im Bereich von 5 bis 17 Gew.-%, jeweils bezogen auf das Gesamtgewicht der Zusammensetzung, liegt.

## Revendications

1. Utilisation de filtres anti-UV afin d'améliorer la pénétration dermique de resvératrol dans des compositions cosmétiques topiques, dans laquelle les filtres anti-UV sont l'octocrylène, et la 1-(4-tertio-butylphényl)-3-(4-méthoxyphényl)-propane-1,3-dione.

2. Utilisation selon la revendication 1, dans des compositions cosmétiques topiques destinées au blanchissement de la peau.

3. Utilisation selon l'une quelconque des revendications précédentes, dans laquelle la composition cosmétique comprend en outre un polyol choisi dans le groupe constitué par le sorbitol, le butylène glycol, le glycérol, le polypropylène glycol, le polyéthylène glycol, le PEG-18, le PPG-18, la diméthicone, l'huile de ricin hydrogénée PEG-40, le stéarate de PEG-20, le sesquistéarate de méthylglucose PEG-20, le dioléate de méthylglucose PEG-120, le cétéareth-12, le coceth-7, le lauryl glycol éther de PPG-1-PEG-9, le stéarate de glycérol PEG-30, le cocoate de glycérol PEG-7 et l'éthoxyglycol.

4. Utilisation selon la revendication 3, dans laquelle le polyol est un polyéthylène glycol.

5. Utilisation selon l'une quelconque des revendications précédentes, dans laquelle la composition cosmétique se trouve sous la forme d'une émulsion H/E ou d'un gel.

6. Utilisation selon l'une quelconque des revendications précédentes, dans laquelle la quantité de resvératrol dans la composition se trouve dans la plage allant de 0,001 à 5% en poids, préférablement dans la plage allant de 0,05 à 2% en poids, tout préférablement dans la plage allant de 0,2 à 1% en poids, chacune par rapport au poids total de la composition.

7. Utilisation selon l'une quelconque des revendications précédentes, dans laquelle la quantité de filtres anti-UV dans la composition se trouve dans la plage allant de 4 à 27% en poids, préférablement dans la plage allant de 6 à 20% en poids, tout préférablement dans la plage allant de 9 à 15% en poids, chacune par rapport au poids total de la composition.

8. Utilisation selon l'une quelconque des revendications 3 à 7, dans laquelle la quantité des un ou plusieurs polyols dans la composition se trouve dans la plage allant de 1 à 30% en poids, préférablement dans la plage allant de 3 à 20% en poids, tout préférablement dans la plage allant de 5 à 17% en poids, chacune par rapport au poids total de la composition.
